# EUROPEAN PATENT SPECIFICATION

(11) **EP 0 630 888 B1**
(45) Date of publication and mention of the grant of the patent: **04.04.2001**
(21) Application number: 93901552.5
(22) Date of filing: 14.01.1993
(51) Int. Cl.: C07D 233/06, A61K 31/415

(54) **IMIDAZOLINE DERIVATIVE, PRODUCTION THEREOF, AND BLOOD PRESSURE RETENTIVE**
IMIDAZOLINDERIVATE, IHRE HERSTELLUNG UND BLUTDRUCKHALTENDE MITTEL
DERIVE D'IMIDAZOLINE, SA PRODUCTION, ET AGENT DE RETENTION DE LA TENSION ARTERIELLE

(43) Date of publication of application: 28.12.1994
(73) Proprietor: Maeda, Hiroshi, Kumamoto-shi Kumamoto (JP)
(72) Inventor: MAEDA, Hiroshi, Kumamoto-shi Kumamoto 862 (JP); MIYAMOTO, Yoichi, Ibaraki 305 (JP); AKAIKE, Takaaki, Kumamoto 862 (JP)
(74) Representative: DIEHL GLAESER HILTL & PARTNER
(86) International application number: JP9300044
(87) International publication number: WO9415921

(56) References cited:
- BIOCHEMISTRY, vol.32, no.3, 26 January 1993, EASTON, PA US pages 827 - 832 T. AKAIKE ET AL 'Antagonistic action of imidazolineoxyl N-oxides against endothelium-derived relaxing factor/.no through a radical reaction'
- CHEMICAL ABSTRACTS, Vol. 117, No. 26 (1992), Abstract No. 263198n.
- CHEMICAL ABSTRACTS, Vol. 68, No. 25 (1968), Abstract No. 114500x.
- CHEMICAL ABSTRACTS, Vol. 109, No. 25 (1988), Abstract No. 229937c.
- CHEMICAL ABSTRACTS, Vol. 117, No. 20 (1992), Abstract No. 192473k.
- CHEMICAL ABSTRACTS, Vol. 81, No. 2 (1974), Abstract No. 8436q.
- CHEMICAL ABSTRACTS, Vol. 117, No. 14 (1992), Abstract No. 142068m.
- CHEMICAL ABSTRACTS, Vol. 117, No. 10 (1992), Abstract No. 90907n.
- CHEMICAL ABSTRACTS, Vol. 115, No. 14 (1991), Abstract No. 148610n.
- CHEMICAL ABSTRACTS, Vol. 114, No. 12 (1991), Abstract No. 102919w.
- CHEMICAL ABSTRACTS, Vol. 112, No. 6 (1990), Abstract No. 36501j.
- CHEMICAL ABSTRACTS, Vol. 115, No. 16 (1991), Abstract No. 173360p.
- CHEMICAL ABSTRACTS, Vol. 95, No. 7 (1981), Abstract No. 61142f.
- CHEMICAL ABSTRACTS, Vol. 80, No. 16 (1974), Abstract No. 83850c.
- CHEMICAL ABSTRACTS, Vol. 116, No. 12 (1992), Abstract No. 119311f.

## Description

The present invention relates to novel imidazoline derivatives, a method for producing the same and a highly safe therapeutic agent for maintaining blood pressure having an imidazoline derivative as the active ingredient and intended for maintaining blood pressure of man for the treatment of shock by means of removing excess nitric oxide (hereinafter referred to as "N0") which is the very substance constituting the endothelium-derived relaxing factor (hereinafter referred to as "EDRF"), which causes relaxation of the vascular smooth muscles.

In the past it was well known that the vascular endothelial cells secrete a substance contributing to relaxation of the vascular smooth muscles, i. e. EDRF. (Ref. Nature, Vol. 288, 373-376, 1980). After a while it was substantiated that NO produced from L-arginine is the very substance which constitutes EDRF. (Ref. Nature, Vol. 327, 524-526, 1987; and Proc. Natl. Acad. Sco., USA, Vol. 84, 9265-9259, 1987).

There exist as N0-synthesizing enzymes (N0 synchase) produced by the endothelial cells the following two kinds of enzymes: (1) the endogenous type which is always present in the vascular endothelium (Ref. Pharm. Rev., Vol. 43, 109-142, 1991; The Lancet, Vol. ii, 997-1000. 1989) and (2) the type which is derived from endotoxin or cytokines such as tumor necrosis factor (TNF)(Ref. Pharm. Rev., Vol. 43, 109-142, 1991). While the former type (1) is thought to contribute to physiological adjustment of the vascular resistance, the abrupt progress of the synthesis of N0 due to the latter type (2) is considered to cause the decline in the vascular resistance and the accompanying fall in blood pressure, presumably causing shock. Most of the patients in such syndrome do not respond to the vasopressor such as norepinephrine and dopamine and fail to get away from the mortal condition.

Various therapies have been studied to cope with such shock as described above caused by endotoxin or cytokines. One of those therapeutic approaches ever experimented was intended to treat laboratory animals in endotoxin shock or patients in septic shock, using L-arginine analogue which is an N0 synthase inhibitor. (Ref. The Lancet, Vol. 338, 1555-1557, 1991, and the Lancet, Vol. 338, 1557-1558, 1991). Since the increasing production of EDRF (namely, N0) is the conclusive and universal phenomenon which occurs with the fall in blood pressure of the patient in endotoxin shock, the effort to decease the amount of N0 is an excellent therapeutic approach to treat endotoxin shock or similar cases. The advantage of the therapeutic agent capable of neutralizing the action of N0 by itself is ever so obvious in consideration of the complexity involved in the therapy to neutralize the action of endotoxin or individual cytokine.

Nevertheless, there have been presented reports to the effect that caution must be taken in determining the dosage to be administered, since the effect of this N0 synthase inhibitor to maintain blood pressure when administered for treatment of shock strongly depends on the dosage such that if it is too little, its efficacy is totally lost, and, conversely, if the dosage is excessive, the patient is led to an extremely dangerous condition as is induced by violent rise and fall in the blood pressure. (Ref. The Lancet, Vol. 338, 1555-1557, 1991; The Lancet, Vol. 338, 1557-1558, 1991) In view of the significance of the aforementioned reports, there still remain unresolved problems associated with safety of the N0 synthase inhibitor. Moreover, since the N0 synthase inhibitor is an L-arginine derivative, there is a possibility that this inhibitor adversely affects L-arginine-dependent protein synthesizing systems other than the N0 synthase as well as metabolic systems such as the (Krebs-Henseleit) urea cycle. In this sense, it is feared that the administration of this N0 synthase inhibitor can be accompanied by some side effects. In particular, it will conceivably give rise to many unknown questions if such inhibitor is administered in a copious amount over an extensive period of time.

The present inventors have studied a method for stably maintaining blood pressure by removing excess N0 produced, for the purpose of resolving the aforementioned problem, without administering any N0 synthase inhibitor.

Meanwhile, although there have been reported a method for producing cigarette filter impregnated with 2-phenyl-4, 4, 5, 5-tetramethylimidazoline-1-oxyl-3-oxide (hereinafter referred to as "PTI0"), which is represented by the general formula cited hereinafter with R² indicated therein being hydrogen atom, for the purpose of removing N0 from cigarette smoke (Ref. B. P. 1235880), and another method which employs PTIO as the N0 oxidizing agent in the instrument to determine nitrogen oxides (N0 and N0₂) in the atmosphere (Ref. "Kankyo To Sokutei Gijutsu" (Environment and Measuring Technology), Vol. 12, 32-39, 1985), it is not known that compounds represented by the general formula (II), including PTI0, ever exhibits an efficacy to maintain blood pressure.

The present inventors, as the result of their study on the efficacy of PTI0 and its derivatives as a therapeutic agent to maintain blood pressure, have discovered those various novel PTI0 derivatives represented by the following general formula (I) with the phenyl group of PTI0 being substituted by a specific substituent and the method for producing the same, and also that the PTI0 represented by the general formula (II) and a component containing the above-mentioned novel or known PTI0 derivates impart the effectiveness to a therapeutic agent to maintain blood pressure, and thus finally completed the present invention.

The object of the present invention is, therefore, to provide an effective and safe therapeutic agent to maintain blood pressure with novel or known compounds having the efficacy as a therapeutic agent to maintain blood pressure possessing a new mechanism of action of eliminating N0.

That is to say, the present invention comprises imidazoline derivatives represented by the general formula (I) 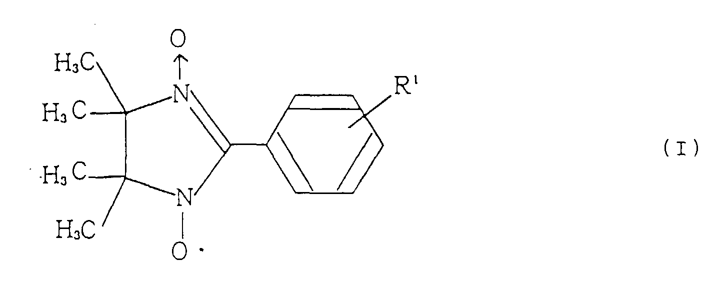 where R¹ means the carboxyl group or the carboxymethoxy group, or a pharmaceutically acceptable salt thereof, and comprises a therapeutic agent for maintaining blood pressure which contains as its active ingredient an imidazoline derivative represented by the following general formula (II) 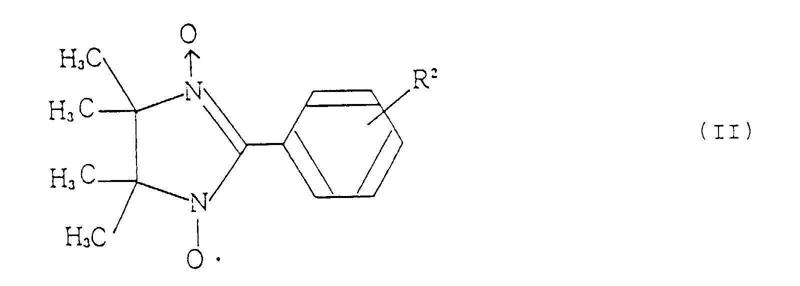 where R² is a hydrogen atom, the carboxyl group or the carboxymethoxy group, or a
pharmaceutically acceptable salt thereof.

As the pharmaceutically acceptable salt, sodium salt or potassium salt is particularly preferable.

As for the synthesis of PTI0 represented by the general formula (II) with R² as indicated therein being hydrogen atom, it is well known that it is obtained by reacting 2,3-bis-(hydroxyamino)-2,3-dimethylbutane with benzaldehyde. (Ref. J. Am. Chem. Soc., Vol. 90, 1078-1079, 1968).

On the other hand, the novel imidazoline derivative represented by the general formula (I) can be produced by reacting 2,3-bis-(hydroxyamino)-2, 3-dimethylbutane with a benzaldehyde derivative.

For example, 2-(4-carboxyphenyl)-4, 4, 5, 5-tetramethylimidazoline-1-oxyl-3-oxide (hereinafter referred to as "C-PTI0") represented by the general formula (I) with R¹ as indicated therein being carboxyl group or a pharmaceutically acceptable salt thereof can be obtained by reacting 2, 3-bis- (hydroxyamino)-2, 3-dimethylbutane with p-formylphenoxybenzoic acid.

Additionally, 2-(4 -carboxymethoxylphenyl)-4, 4, 5, 5-tetramethylimidazoline-1-oxyl-3-oxide (hereinafter referred to as "CM-PTI0") represented by the general formula (I) with R¹ as indicated therein being carboxymethoxyl group or a pharmaceutically acceptable salt thereof can be obtained by reacting 2, 3-bis-(hydroxyamino)-2, 3 -dimethylbutane with p-formylphenoxyacetic acid.

The PTI0 and the imidazoline derivativeshaving the substituent indicated in the general formula (I) (hereinafter collectively referred to as "PTI0 and its derivatives") which are thus produced can be identified by ¹ H-nuclear magnetic resonance (NMR), infrared absorption spectrometry, mass spectrometry or electron spin resonance (ESR) analysis of the obtained product and intermediates thereof.

The therapeutic agent to maintain blood pressure disclosed by the present invention may be utilized as a composition containing effective and low toxicity contents. The effective and low-toxicity dosage is 5-500 mg kg-weight day.

The therapeutic agent to maintain blood pressure disclosed by the present invention may be administered in the form of injection, oral dosage, suppository or aerosol spray.

In the case of injection, any of the subcutaneous, intramuscular, intravenous, and intra-arterial routes is workable. It is also possible to infuse it by continuous intravenous infusion. In the case of injection, although there is not imposed any limitation on the concentrations of PTI0 and its derivatives, it is preferable that the concentrations of PTI0 and its derivative be 0.001-5% by weight. It is also possible that PTI0 and its derivatives, both being prepared in powder form, are combined with a liquid solvent to be administered as an injection.

The type of preparation for oral administration may be selected optionally. Some examples of this type are tablet, granules, pill, liquid medicine, syrup, troche , and drops. All of the said preparations may be manufactured conventionally. Furthermore, PTI0 and its derivativesmay be suspended in oil so that enteral absorption may be enhanced.

PTI0 and its derivativescan be formed by the conventional method into suppository for anal or vaginal application. Aerosol spray preparation may be manufactured conventionally, using a suitable propellant.

PTI0 and its derivativesreact with N0 at the molar ratio of 1 to 1, and are converted, with one oxygen atom added thereto, into nitrogen dioxide (hereinafter referred to as "NO₂") which is inactive to vascular smooth muscular cells. N0₂ is, furthermore, converted into nitrous ion and nitrate ion in aqueous solution. The present inventors have discovered that PTI0 and its derivatives themselves are converted into substances inactive to N0, that PTI0 and its derivativesforcefully suppress relaxation of the N0-dependent vascular smooth muscles, and also that the blood pressure and the pulse rate of animals given PTI0 and its derivativesstably remained at normal levels even if N0-saturated aqueous solution was infused into their blood vessels. As has been discussed, PTI0 and its derivatives do not obstruct the synthesis of N0, but eliminates excess N0 produced. Hence, they do serve as a highly safe therapeutic agent to maintain blood pressure.

The present invention shall be explained in detail with the following examples:

### EXAMPLE 1:Synthesis of 2-(4-carboxyphenyl)-4, 4, 5, 5-tetramethylimidazoline-1-oxyl-3-oxide (C-PTI0)

24.6 g of 2,3-bis-(hydroxyamino)-2,3-dimethylbutane sulfate was dissolved in 100 ml of water. After neutralized with 1 M KHC0₃ with cooling on ice, the solution was mixed with 15.0 g of p-formylbenzoic acid and was agitated overnight at room temperature. The precipitate was dried and 16.9 g of 1,3-dihydroxy-4, 4, 5, 5-tetramethyl 2-(4-carboxyphenyl)imidazole (I) was obtained.

After the said intermediate (I) was subjected to observation by ¹H-NMR, and fast atomic bombardment mass spectrometry (FAB-MS) for identification, the sample was verified to be the above-mentioned intermediate (I) with the following test results:
(1) ¹H-NMR (DMS0 -d₆)
   δ : -7.73 (dd, 4 H, Ar-H)
   4.57 (s, 1H, C-H)
   4.10 (broad s, 2 H, NOH)
   1.05 (s, 6 H, CH₃)
   1.09 (s, 6 H, CH₃)
(2) FAB-MS
   m/z 279 (M-H)⁻

Next, 14.0 g of (I) was dissolved in 100 ml of N,N-dimethylformaldehyde, and the solution was mixed with 23.5 g of Pb0₂ to succeedingly undergo reaction for 3 hours at room temperature. Then, Pb0₂ was filtered out. After the concentrated residue of the filtrate was dissolved in water, the solution was freeze-dried and thus wereobtained 12.5 g of C-PTI0. By infrared absorption spectrometry, mass spectrometry, 1H-nuclear NMR and ESR, the product thus obtained was identified to be as follows:
(1) IR (KBr disk): 1360 cm⁻¹ (N-0)
(2) FAB-MS
   m/z 276 (M-H)⁻
(3) 1H - NMR (D₂0)
   δ : 2.90 (d, 12H, CH₃)
   7.90 (s, 4 H, Ar-H)
(4) ESR (0.25 M phosphoric acid buffer, pH7.5)
   a_{N}^{1.3}= 0.81 mT

On the basis of the above-mentioned test results, it was verified that the product is 2-(4-carboxy-phenyl)-4, 4, 5, 5-tetramethylimidazoline-l-oxyl 3-oxide (C-PTI0).

### EXAMPLE 2:Synthesis of 2-(4-carboxy-methoxyphenyl)-4,4,5,5-tetramethylimidazoline-1-oxyl-3-oxide (CM-PTI0)

24.6 g of 2,3-bis-(hydroxyamino)-2,3-dimethylbutane sulfate was dissolved in 100 ml of water. After the solution was neutralized with 1 M KHC0₃ with cooling in ice, it was mixed with 18.0 g of p-phenoxyacetic acid, and was agitated overnight at room temperature. Then, the produced precipitate was dried, and was dissolved in 100 ml of N, N-dimethylformaldehyde. After 45 g of Pb0₂ was added, the liquid underwent reaction for 3 hours at room temperature. After filtering out Pb0₂, the filtrate was concentrated. The residue was dissolved in water, and this aqueous solution was freeze-dried to obtain 15.1 g g of CM-PTI0. The analytical datas thus determined are as follows:
(1) IR (KBr disk): 1360 cm⁻¹ (N-0)
(2) FAB-MS
   m/z 306 (M-H)⁻
(3) 1H-NMR(D₂0)
   δ : 1.35 (d, 12 H, CH₃)
   1.65 (s, 2 H, CH₂)
   7.10 (d, 2 H, Ar-H)
   8.05 (d, 2 H, Ar-H)
(4) ESR (0.25 M phosphoric acid buffer, pH 7.5)
   a_{N}^{1.3} = 0.82 mT

On the basis of these test results, the product was identified to be 2-(4-carboxymethoxyphenyl)-4, 4, 5, 5-tetramethylimidazoline-1-oxyl-3-oxide (CM-PTI0).

### EXAMPLE 3: Production of C-PTI0 injection

C-PTI0 was dissolved in 800 ml of distilled water for injection use. After adding sodium chloride to this aqueous solution, an injection preparation of 100 ml total volume was obtained by adding distilled water for injection as needed. After sterilizing the solution by letting it pass through a bacterial filter, a C-PTIO injection preparation having the following ingredients was obtained:

| | |
|---|---|
| C-PTI0 | 2.0 g |
| Sodium chloride | 9.0 g |
| Distilled water for injection | Appropriate volume |
| Total volume | 1,000 ml |

### EXAMPLE 4: Production of PTI0 preparation

PTI0, lactose and starch were mixed and formed into wet granules with an aqueous solution of polyvinylpyrrolidone. After the obtained material was dried and sifted, the granules were kneaded with magnesium stearate and compressed into tablets, each of which weighing 500 mg and consisting of the following ingredients:

| | |
|---|---|
| PTI0 | 100 weight parts |
| Lactose | 235 " " |
| Starch | 50 " " |
| Polyvinylpyrrolidone | 50 " " |
| Magnesium stearate | 5 " " |

### EXAMPLE 5:Production of CM-PTI0 suppository

The following ingredients were melt-mixed and formed into 10 pieces of suppositories:

| | |
|---|---|
| CM-PTI0 | 5.0 g |
| Macrogol 4000 (polyethyleneglycol) | 2.0 g |
| Macrogol 1500 (polyethyleneglycol) | 9.0 g |

### EXAMPLE 6: Suppression of EDRF-dependent relaxation (dilation) of vascular smooth muscles by therapeutic agent to maintain blood pressure

Rabbit aorta with vascular endothelium unremoved was cut into the ring-form and was hung in an organ bath filled with Krebs-Ringer's solution and its tension was continuously recorded. After the rabbit aorta was contracted by exposure to 0.15 microM phenylephrine, it was completely dilated by adding 3 microM acetylcholine. It is well known that the dilation of the vascular smooth muscle by acetylcholine is dependent on EDRF, namely N0. The degree of PTI0's ability to suppress dilation caused by acetylcholine was determined by adding C-PTI0 or CM-PTI0 prepared in EXAMPLES 1-2 in such manners that the concentration would be registered at 30 microM, 100 microM and 300 microM, respectively. Used as a reference was N^{G}-methyl-arginine, which is a N0 synthase inhibitor, at the corresponding concentrations. The test result is shown in Table 1.

**Table 1.**

| The ability to suppress EDRF-dependent relaxation (dilation) of vascular smooth muscles of PTIO, C-PTIO, CM-PTIO and N^{G}-methylarginine | | | | |
|---|---|---|---|---|
| | Percentage of suppression against relaxation of vascular smooth muscles | | | |
| Concentration (microM) | PTIO | C-PTIO | CM-PTIO | N^{G}-methylarginine |
| 0 | 0 | 0 | 0 | 0 |
| 30 | 25.1 ± 2.0 | 47.2±5.5 | 26.5 ± 1.9 | 21.3±3.2 |
| 100 | 52.4 ±4.6 | 72.5±2.0 | 46.5±1.8 | 48.7 ± 9.5 |
| 300 | 78.5±1.3 | 93.0±1.8 | 84.5 ± 6.6 | 74.5±5.0 |
| Note 1: The vascular smooth muscles which were contracted by 0.15 microM phenylephrine were relaxed (dilated) by 100% by 3.0 miroM acetylcholine. The percentage of suppression against relaxation was given the rating of 0%. Note 2: Each measurement represents the mean average of 10 test results plus minus standard deviation. As can be obviously seen from Table 1, PTI0, C-PTI0, and CM-PTI0 suppressed the EDRF-dependent relaxation of vascular smooth muscles, concentration-dependently. The intensity of suppression effected by PTI0 and CM-PTI0 was either equivalent to or somewhat more intensive than that of N^{G}-methyl-arginine. The intensity of suppression by C-PTI0 was twice stronger than the said substances. | | | | |

### EXAMPLE 7: The effect of PTI0's suppression against the decline in blood pressure caused by intravenous infusion of aqueous solution of N0

Tail veins of rats anesthetized with Nembutal (pentobarbital sodium) were cannulated to establish the route for intravenous administration of therapeutic agent. The average blood pressure and the pulse rate were monitored on the basis of the pulse pressure wave of the tail artery, using an automatic blood pressure measuring apparatus (PS-200) manufactured by Riken Kaihatsu Company, Limited.

2.0 mg/ml of PTI0 injection prepared according to the manufacturing example 1 was administered to the first group of rats by continuous intravenous infusion at the rate of 6.0 ml/hour for 10 minutes. After the lapse of 4 minutes from then, 1.0 ml of N0-saturated physiological solution of sodium chloride was continuously infused intravenously. For the second group of rats, PTI0 was not administered, but N0 was administered similarly instead. Except for the period in which the said dosage was administered, physiological solution of sodium chloride was administered by continuous intravenous infusion at the rate of 6.0 ml/hour. All the while, the blood pressure and the pulse rate were continuously monitored, and additionally the ratio of blood pressure and pulse rate recorded five minutes after the administration of the dosage to those recorded five minutes prior to the administration of the same was calculated. The test result is shown in Table 2.

**Table 2**

| The effect of PTIO on changes in blood pressure and pulse rate caused by administration of NO | | |
|---|---|---|
| Specimen Group | Blood pressure (% to prior to administration of NO) | Pulse rate (% to prior to administration of NO) |
| 1 | 93.1 ± 2.3 | 96.5 ± 6.4 |
| 2 | 55.8 ± 1.8 | 126.3 ± 2.0 |
| LEGEND: "Specimen Group 1": A group of 3 rats to which 2 mg/ml of PTI0 was administered at the rate of 6 ml/hour for 10 minutes. "Specimen Group 2": A reference group of 3 rats to which PTI0 was not administered. The measurement represents the average plus minus standard deviation. | | |

According to Table 2, although the blood pressure dropped about 40% and the pulse rate increased about 20% in consequence of the administration of N0-saturated physiological solution of sodium chloride (Specimen Group 2), the changes in blood pressure and pulse rate were almost nil if PTI0 had been administered beforehand. (Specimen Group 1)

The present invention is to provide a therapeutic agent to maintain blood pressure for the treatment of shock. High safety is achieved in the therapeutic use of this agent intended to maintain blood pressure in the treatment of shock due to the fall in blood pressure caused by burns, septicemia, stings by bees, wasps and hornets, snakebite, anaphylaxis, poisoning caused by organophosphorous agents, or side effect of various cytokine therapies. It is also possible to use it as an efficacy accelerant for cardiacs.

## Claims

1. The imidazoline derivatives represented by the following general formula (I) 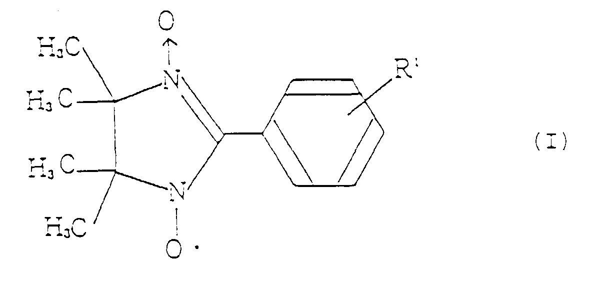 wherein R¹ means the carboxyl group or the carboxymethoxy group, and pharmaceutically acceptable salts thereof.

2. A process for producing the imidazoline derivatives according to claim 1, characterized in that 2,3-bis-(hydroxyamino)-2,3-dimethylbutane is reacted with formylbenzoic acid.

3. A process for producing the imidazoline derivatives according to claim 1, characterized in that 2,3-bis-(hydroxyamino) -2,3-dimethylbutane is reacted with formylphenoxy acetic acid.

4. A therapeutic agent for maintaining blood pressure, characterized in that it contains as its active ingredient an imidazoline derivative represented by the general formula (II) 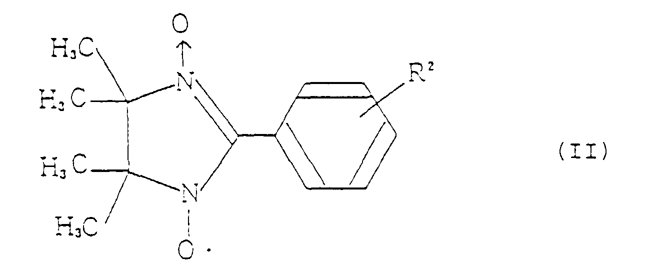 wherein R² means a hydrogen atom, the carboxyl group or the carboxymethoxy group, and pharmaceutically acceptable salts thereof.

5. The therapeutic agent according to claim 4, characterized in that its active agent is 2-phenyl-4,4,5,5-tetramethylimidazoline-1-oxyl-3-oxide.

6. The therapeutic agent according to claim 4 or 5, characterized in that the pharmaceutically acceptable salt is the potassium or sodium salt.

## Patentansprüche

1. Imidazolinderivate der folgenden allgemeinen Formel (I) 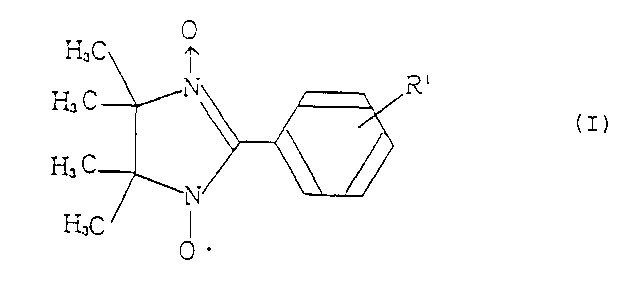 in der R¹ die Carboxylgruppe oder die Carboxymethoxygruppe bedeutet, und pharmazeutisch annehmbare Salze hiervon.

2. Verfahren zum Herstellen der Imidazolinderivate nach Anspruch 1, dadurch gekennzeichnet, daß 2,3-Bis- (hydroxyamino)-2,3-dimethylbutan mit Formylbenzoesäure umgesetzt wird.

3. Verfahren zum Herstellen der Imidazolinderivate nach Anspruch 1, dadurch gekennzeichnet, daß 2,3-Bis-(hydroxyamino) -2,3-dimethylbutan mit Formylphenoxyessigsäure umgesetzt wird.

4. Therapeutisches Mittel zum Aufrechterhalten des Blutdrucks, dadurch gekennzeichnet, daß es als Wirkstoff ein Imidazolinderivat der allgemeinen Formel (II) 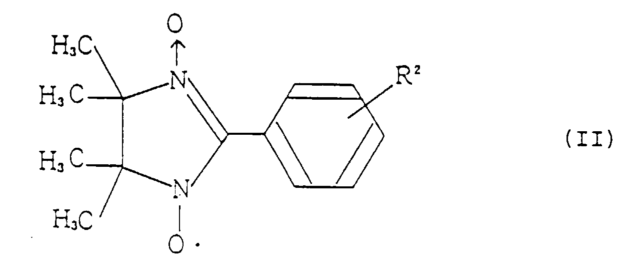 in der R² ein Wasserstoffatom, die Carboxylgruppe oder die Carboxymethoxygruppe bedeutet, und pharmazeutisch annehmbare Salze hiervon enthält.

5. Therapeutisches Mittel nach Anspruch 4, dadurch gekennzeichnet, daß sein Wirkstoff 2-Phenyl-4,4,5,5-tetramethylimidazolin-1-oxyl-3-oxid ist.

6. Therapeutisches Mittel nach Anspruch 4 oder 5, dadurch gekennzeichnet, daß das pharmazeutisch annehmbare Salz das Kalium- oder Natriumsalz ist.

## Revendications

1. Dérivés imidazoline représentés par la formule générale suivante (I): 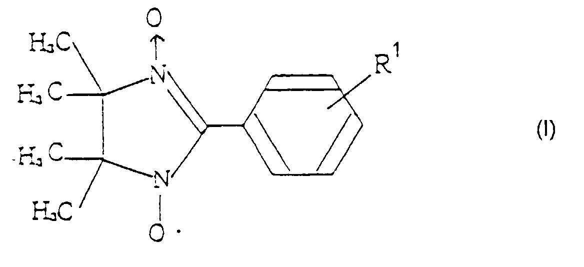 dans laquelle R¹ représente un groupe carboxyle ou un groupe carboxyméthoxy et ses sels acceptables sur le plan pharmaceutique.

2. Procédé pour produire les dérivés imidazoline selon la revendication 1, caractérisé en ce que l'on fait réagir le 2,3 -bis- (hydroxyamino)-2,3-diméthylbutane avec l'acide formylbenzoïque.

3. Procédé pour produire les dérivés imidazoline selon la revendication 1, caractérisé en ce que l'on fait réagir le 2,3-bis- (hydroxyamino)-2,3-diméthylbutane avec l'acide formylphénoxyacétique.

4. Agent thérapeutique pour le maintien de la pression sanguine, caractérisé en ce qu'il contient, en tant que principe actif, un dérivé imidazoline représenté par la formule générale (II): 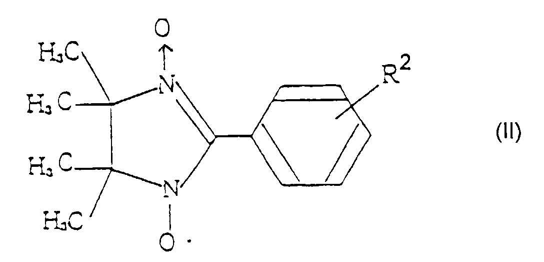 dans laquelle R² représente un atome d'hydrogène, un groupe carboxyle ou un groupe carboxyméthoxy et ses sels acceptables sur le plan pharmaceutique.

5. Agent thérapeutique selon la revendication 4, caractérisé en ce que le principe actif est le 2-phényl-4,4,5,5-tétraméthylimidazoline-1-oxyl-3-oxyde.

6. Agent thérapeutique selon la revendication 4 ou 5, caractérisé en ce que le sel d'addition acceptable sur le plan pharmaceutique est un sel de potassium ou un sel de sodium.
